# EUROPEAN PATENT APPLICATION

(11) **EP 4 365 301 A1**
(43) Date of publication of application: **08.05.2024**
(21) Application number: 22832937.1
(22) Date of filing: 21.06.2022
(51) Int. Cl.: C12P 13/00, A01N 47/40, A01P 3/00, A61K 31/277, A61P 31/04, C07C 291/10, C12P 1/06

(54) **COMPOUND, MANUFACTURING METHOD THEREFOR, ANTIMICROBIAL AGENT, AND MEDICINAL DRUG**

(30) Priority: 30.06.2021 JP 2021109057
(71) Applicant: Murata Manufacturing Co., Ltd., Nagaokakyo-shi, Kyoto 617-8555 (JP); National University Corporation Hokkaido University, Sapporo-shi, Hokkaido 060-0808 (JP)
(72) Inventor: TAKATA, Masachika, Nagaokakyo-shi, Kyoto 617-8555 (JP); SUNAHARA, Hirofumi, Nagaokakyo-shi, Kyoto 617-8555 (JP); WAKIMOTO, Toshiyuki, Sapporo-shi, Hokkaido 060-0808 (JP); MATSUDA, Kenichi, Sapporo-shi, Hokkaido 060-0808 (JP)
(74) Representative: Reeve, Nicholas Edward
(86) International application number: PCT/JP2022/024740
(87) International publication number: WO 2023/276789

(57) **Abstract**

A compound represented by the following Formula (1), Formula (2), or Formula (3), or a salt thereof, or a solvate thereof: 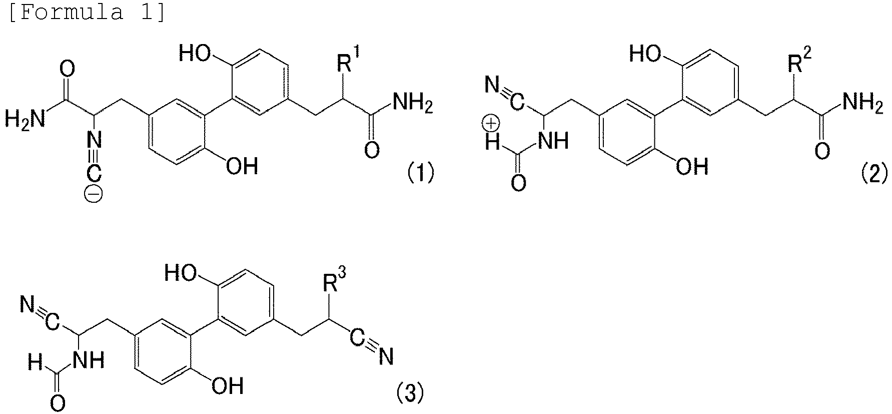 wherein R¹, R², and R³ are the same or different and each represent a hydrazine group, an N-hydroxy-acetylamino group, or an N-hydroxy-formylamino group.

## Description

### TECHNICAL FIELD

The present invention relates to a novel compound having an antimicrobial action and a method for producing the same. The present invention also relates to an antimicrobial agent and a medicine containing the above compound.

### BACKGROUND ART

Physiologically active substances such as antibiotics have been hitherto found from metabolites of actinomycetes. Non-Patent Literature 1 describes that a P07101 strain of actinomycetes belonging to the genus Kitasatospora (Kitasatospora sp.) produced hazimycin A and analogous compounds thereof, and hazimycin A had an antimicrobial activity against gram-positive bacteria.

### CITATION LIST

### - Non-Patent Literature

Non-Patent Literature 1: Acta Pharmaceutica Sinica B Volume 5, Issue 6, 2015, Pages 564 to 568

### SUMMARY OF INVENTION

### - Technical Problem

An object of the present invention is to provide a novel compound having an antimicrobial action and a method for producing the same. Another object of the present invention is to provide an antimicrobial agent and a medicine containing the above compound.

### - Solution to Problem

The compound of the present invention is a compound represented by the following Formula (1), Formula (2), or Formula (3), or a salt thereof, or a solvate thereof: 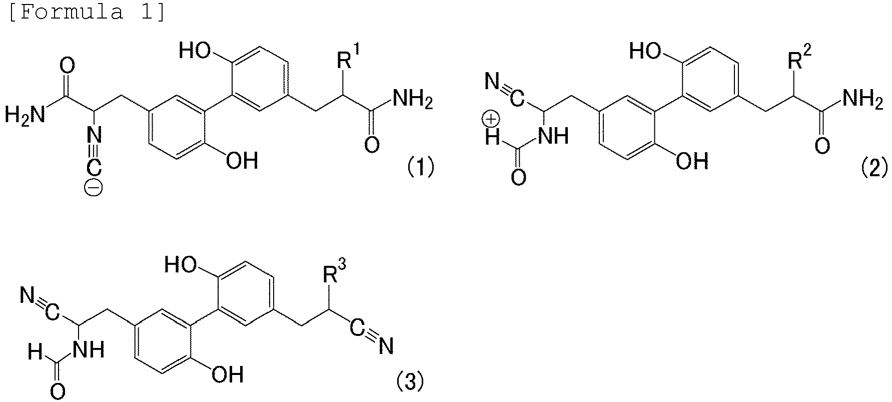 wherein R¹, R², and R³ are the same or different and represent a hydrazine group, an N-hydroxy-acetylamino group, or an N-hydroxy-formylamino group.

The production method of the present invention is a method for producing the compound or a salt thereof, or a solvate thereof, including: a culture step of culturing a Kitasatospora purpeofusca HV058 strain (accession number NITE BP-03475); and a collection step of collecting the compound or a salt thereof, or a solvate thereof from a culture obtained in the culture step.

The antimicrobial agent of the present invention contains the compound or a salt thereof, or a solvate thereof.

The medicine of the present invention contains the compound or a salt thereof, or a solvate thereof.

### - Advantageous Effects of Invention

According to the present invention, a novel compound having an antimicrobial action and a method for producing the same can be provided. According to the present invention, an antimicrobial agent and a medicine containing the compound can be provided.

### DESCRIPTION OF EMBODIMENTS

Hereinafter, a compound of the present invention, a method for producing the same, an antimicrobial agent, and a medicine will be described. Note that the present invention is not limited to the following configuration, and may be appropriately modified without departing from the gist of the present invention. The present invention also includes a combination of a plurality of preferred configurations described below.

### [Compound having antimicrobial action]

The compound of the present invention is a compound represented by the following Formula (1), Formula (2), or Formula (3), or a salt thereof, or a solvate thereof.

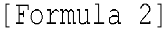

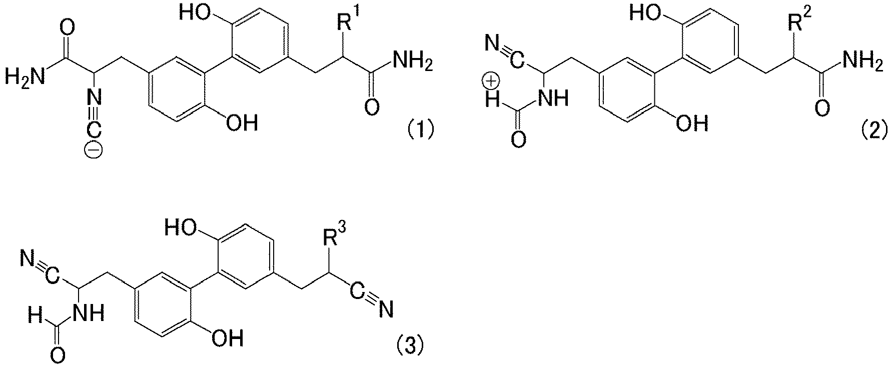

In Formula (1), R¹ represents a hydrazine group, an N-hydroxy-acetylamino group, or an N-hydroxy-formylamino group. R¹ is preferably a hydrazine group (-NH-NH₂).

In Formula (2), R² represents a hydrazine group, an N-hydroxy-acetylamino group, or an N-hydroxy-formylamino group. R² is preferably a hydrazine group.

In Formula (3), R³ represents a hydrazine group, an N-hydroxy-acetylamino group, or an N-hydroxy-formylamino group. R³ is preferably a hydrazine group.

The compound represented by the above Formula (1) is also referred to as a compound (1) in the present specification. The same applies to compounds of other formula numbers, and for example, the compounds represented by Formulae (2) to (3) are also referred to as compounds (2) to (3), respectively.

In addition, hereinafter, the compound represented by the above Formula (1), Formula (2), or Formula (3), or a salt thereof, or a solvate thereof is also referred to as a compound (I).

In the above Formula (1), the carbon atom to which the isonitrile group (-NC) is bonded and the carbon atom to which R¹ is bonded are asymmetric carbons.

In the above Formula (2), the carbon atom to which the nitrile group (-CN) is bonded and the carbon atom to which R² is bonded are asymmetric carbons.

In the above Formula (3), the carbon atom to which the nitrile group is bonded and the carbon atom to which R³ is bonded are asymmetric carbons.

The compound (1), the compound (2), and the compound (3) include any of a compound in which each asymmetric carbon atom has an R configuration, a compound in which each asymmetric carbon atom has an S configuration, and a compound of any combination thereof. In addition, any of racemic compounds, racemic mixtures, single enantiomers, and diastereomeric mixtures thereof is included in the compound of the present invention.

The compound (1), the compound (2), and the compound (3) are each preferably a compound in which the absolute configurations of two asymmetric carbon atoms are both the S configuration (SS) or a compound in which the absolute configurations of two asymmetric carbon atoms are both the R configuration (RR), more preferably a compound in which the absolute configurations of two asymmetric carbon atoms are both the S configuration (SS).

Preferred embodiments of the compound represented by Formula (1) include a compound represented by the following Formula (1a).

Preferred embodiments of the compound represented by Formula (2) include a compound represented by the following Formula (2a).

Preferred embodiments of the compound represented by Formula (3) include a compound represented by the following Formula (3a).

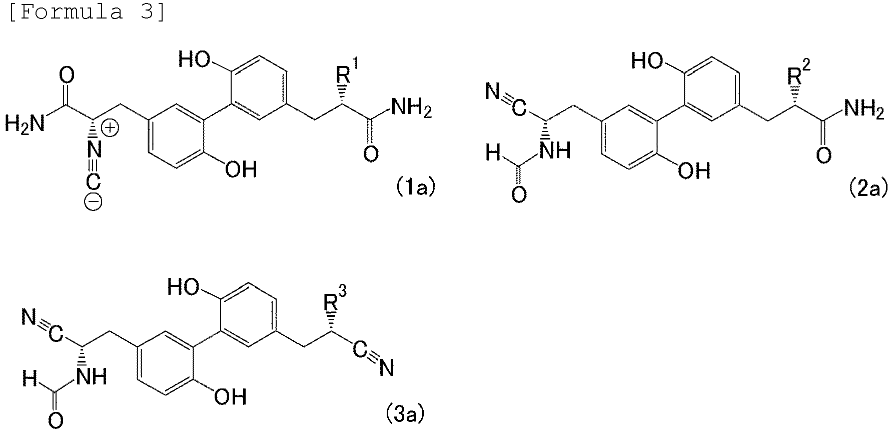

R¹, R² and R³ are the same as described above, preferably a hydrazine group.

The compound (I) of the present invention is preferably a compound represented by Formula (1) or a salt thereof, or a solvate thereof, more preferably a compound represented by Formula (1a) or a salt thereof, or a solvate thereof, still more preferably a compound represented by the following Formula (1a-1) in which R¹ in Formula (1a) is a hydrazine group or a salt thereof, or a solvate thereof.

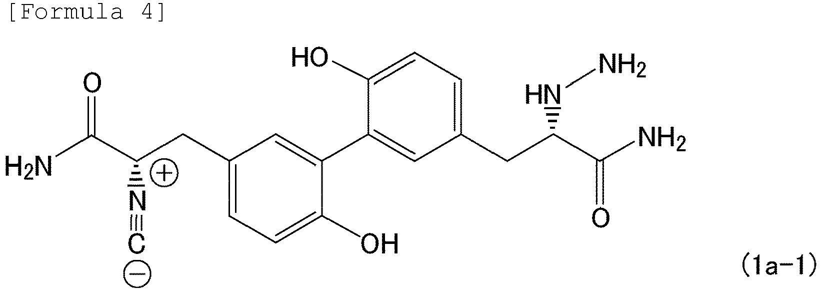

The salt of the compound is not limited as long as it is a pharmaceutically acceptable salt. As the salt, either an acidic salt or a basic salt can be employed. Examples of the acidic salt include inorganic acid salts such as hydrochloride, sulfate, nitrate, hydrofluoride, hydrobromide, and phosphate; and organic acid salts such as acetate, tartrate, lactate, citrate, fumarate, maleate, malate, succinate, methanesulfonate, ethanesulfonate, benzenesulfonate, p-toluenesulfonate, naphthalenesulfonate, and camphorsulfonate. Examples of the basic salt include alkali metal salts such as sodium salts and potassium salts; alkaline earth metal salts such as calcium salts and magnesium salts; salts with ammonia; and salts with organic amines such as morpholine, piperidine, pyrrolidine, monoalkylamine, dialkylamine, trialkylamine, mono(hydroxyalkyl)amine, di(hydroxyalkyl)amine, and tri(hydroxyalkyl)amine.

The solvate of the compound or a salt thereof is not limited as long as it is a solvate of the compound or a salt thereof, and a solvent. Examples of the solvent include water; alcohols such as ethanol and glycerol; and acetic acid. Among them, the solvate is preferably for example, a hydrate and an alcoholate, more preferably a hydrate.

### [Production method]

The method for producing the compound (I) of the present invention is not limited, and can be appropriately selected. The compound (I) of the present invention can be produced, for example, by a method including a culture step of culturing a Kitasatospora purpeofusca HV058 strain (accession number NITE BP-03475), and a collection step of collecting the compound or a salt thereof, or a solvate thereof from a culture obtained in the culture step.

A method for producing the compound or a salt thereof, or a solvate thereof, the method including the culture step and the collection step is also one of the present invention. The compound represented by the above Formula (1), Formula (2), or Formula (3), or a salt thereof, or a solvate thereof can be produced by the production method of the present invention.

The production method of the present invention may include steps other than the culture step and the collection step as necessary.

The Kitasatospora purpeofusca HV058 strain (HV058 SIID. 34879-01) (hereinafter, simply described as HV058 strain) has been deposited at the National Institute of Technology and Evaluation, Patent Microorganisms Depositary (292-0818, #122, 2-5-8, Kazusakamatari, Kisarazu-shi, Chiba, Japan) (accession date: 23, April, 2021, accession number: NITE BP-03475). The HV058 strain is an actinomycete belonging to the genus Kitasatospora. The HV058 strain can produce at least one of the compounds (I). The HV058 strain is suitably used, for example, for producing a compound represented by the above Formula (1a-1).

The culturing in the culture step can be performed according to a known method used for culturing actinomycetes.

The medium is not limited as long as the HV058 strain can grow and the target substance is produced. As the medium, a known medium which is usually used as a medium for actinomycetes belonging to the genus Kitasatospora can be used as it is, or a medium modified as appropriate can be used. As the medium, either a liquid medium or a solid medium can be used, but a liquid medium is preferably used.

As the medium, for example, a medium containing a component selected from a carbon source, a nitrogen source, other inorganic salts, and the like as necessary can be used.

Examples of the carbon source include glucose, sucrose, lactose, galactose, mannose, and glycerol. As the carbon source, one or a combination of two or more thereof can be used. The carbon source is preferably glucose. The concentration of the carbon source in the medium is preferably 0.1 wt% or more and 10 wt% or less, more preferably 1 wt% or more and 5 wt% or less.

Examples of the nitrogen source include peptone, yeast extract, meat extract, soybean flour, ammonium sulfate, and urea. As the nitrogen source, one or a combination of two or more thereof can be used. The concentration of the nitrogen source in the medium is preferably 0.1 wt% or more and 10 wt% or less, more preferably 1 wt% or more and 5 wt% or less.

The concentrations of the carbon source and the nitrogen source may be the above concentrations at the start of culturing.

Examples of other components include inorganic salts such as dipotassium hydrogen phosphate, potassium phosphate, magnesium sulfate, zinc sulfate, iron, manganese, molybdenum, sodium chloride, potassium chloride, and magnesium chloride. One or a combination of two or more thereof can be used.

In one embodiment, a porous adsorbent is preferably added to the medium. Addition of the porous adsorbent to the medium results in faster growth of the HV058 strain. As a result, the production amount of the compound (I) in the culture increases. The porous adsorbent adsorbs the compound (I) produced in the culturing. Therefore, the purification of the compound (I) can be easily performed by separating the porous adsorbent from the obtained culture after culturing.

Examples of the porous adsorbent include synthetic adsorbents, ion exchange resins, and activated carbon. Among them, a synthetic adsorbent is preferable. Examples of the synthetic adsorbent include synthetic adsorbent resins such as aromatic resins (for example, a styrene resin such as styrene-divinylbenzene copolymer). Among them, a styrene resin is preferable. As the styrene resin, for example, a commercially available product such as DIAION (trademark) HP20 (manufactured by Mitsubishi Chemical Corporation) can be used.

The amount of the synthetic adsorbent to be used is preferably 1 wt% or more and 10 wt% or less, more preferably 2 wt% or more and 5 wt% or less in the medium.

The pH of the liquid medium is, for example, preferably 6.0 or more and 9.0 or less, more preferably 7.0 or more and 8.0 or less. For the pH adjustment, a known acidic or alkaline substance can be used. The pH is a pH at 25°C.

The culturing may be performed separately for preculture and main culture. The medium used for the preculture is not limited, and may be the same medium as or a different medium from the liquid medium used for the main culture. At the time of culturing, for example, the HV058 strain may be pre-cultured in a liquid medium and then inoculated into the liquid medium for the main culture, or the HV058 strain cultured in a solid medium such as an agar medium may be directly inoculated into the liquid medium.

The culture temperature is not limited as long as it is a temperature at which an actinomycete belonging to the genus Kitasatospora can be cultured. The culture temperature may be, for example, 15°C or higher and 45°C or lower, and is preferably 20°C or higher and 40°C or lower. When the culture temperature is in this temperature range, the growth of the HV058 strain is fast, and the production amount of the compound (I) increases.

The culture time is not limited as long as the production of the compound (I) is possible. The culture time may be, for example, 10 hours or more and 4 weeks or less, preferably 1 day or more and 4 weeks or less, more preferably 5 days or more and 2 weeks or less.

In the culture step, the HV058 strain is preferably aerobically cultured. The method for performing aerobic culture is not limited, and a liquid medium inoculated with the HV058 strain may be subjected to, for example, shaking culture or stirring culture. The speed of shaking or stirring is not limited, and may be, for example, 30 rpm or more and 500 rpm or less, preferably 50 rpm or more and 300 rpm or less. In addition, the culture method may be any of batch culture, fed-batch culture, and continuous culture, but batch culture is preferable. In the production method of the present invention, static culture may be performed.

By the above culturing, the compound (I) can be accumulated in the culture. The culture contains a medium and bacterial cells of the HV058 strain. The compound (I) accumulates in the medium and/or in the bacterial cells of the HV058 strain.

In the collection step, the compound (I) is collected from a culture obtained in the culture step. The compound (I) may be in any form of the above compound or a salt thereof, or a solvate thereof.

The method for collecting the compound (I) is not limited, and a known method used for separation and purification of a compound can be employed.

When the compound (I) accumulated in bacterial cells is collected, for example, bacterial cells are disrupted by ultrasonic waves, autodigestion, or the like, and bacterial cell residues are removed from the resulting bacterial cell disrupted product by centrifugation, filtration, or the like to obtain a supernatant. The compound of the present invention can be obtained by further purifying the supernatant of the obtained bacterial cell disrupted product. The purification can be performed by, for example, a known separation and purification method such as separation with a porous adsorbent such as a synthetic adsorbent, reverse phase high performance liquid chromatography, or chromatography using an ion exchange resin, alone or in appropriate combination.

The method for separating the bacterial cells and the medium is not limited, and a known method such as centrifugation or filtration can employed.

When the compound (I) is collected from the medium, the compound (I) can be collected by extraction from the medium after culturing, using an organic solvent such as methanol or ethyl acetate. In addition to the above extraction method, the compound (I) can be purified by performing a known separation and purification method such as separation by a porous adsorbent such as a synthetic adsorbent, reverse phase high performance liquid chromatography, chromatography using, for example, ion exchange resin, alone or in appropriate combination.

When the porous adsorbent described above is added to the medium and culturing is performed, the compound (I) is adsorbed to the adsorbent. Therefore, the compound (I) can be collected from the culture by recovering the porous adsorbent from the culture. An extract containing the compound (I) can be obtained by extracting the porous adsorbent using, for example, an organic solvent such as methanol or ethyl acetate, or a mixed solution of the organic solvent and water. The compound (I) can be purified by subjecting this extract to the above known separation and purification methods alone or in appropriate combination.

The compound (I) obtained by the production method of the present invention may be one type or a mixture of two or more types of compounds.

The compound represented by the above Formula (1), Formula (2), or Formula (3), or a salt thereof, or a solvate thereof can also be produced by a known chemical synthesis method. In addition, for example, the compound (I) can also be obtained by using, as a starting substance, a compound obtained by culturing the HV058 strain described above (for example, a compound represented by the above Formula (1a-1)), and subjecting the compound to a known synthesis reaction or a synthesis reaction according to the known synthesis reaction. The target compound obtained by synthesis can be purified by an ordinary purification method such as reverse phase high performance liquid chromatography, and chromatography using, for example, an ion exchange resin or a synthetic adsorbent resin.

Whether the desired compound is obtained can be confirmed by a known method (for example, mass spectrometry, nuclear magnetic resonance spectroscopy (NMR), or high-performance liquid chromatography (HPLC)).

### [Applications of compound]

The compound represented by the above Formula (1), Formula (2), or Formula (3), or a salt thereof, or a solvate thereof has an antimicrobial action. Therefore, the compound represented by the above Formula (1), Formula (2), or Formula (3), or a salt thereof, or a solvate thereof can be used as, for example, an antimicrobial agent or a medicine.

The compound (I) can be used alone or in the form of a composition obtained by blending with other components, in various fields such as medicines (including quasi-drugs), foods and beverages (including general foods and beverages, health foods, foods labeled with functionality, foods for specified health uses, dietary supplements, nutritional supplements, foods and beverages for patients, and food additives, for example), reagents, cosmetics, and daily necessities.

An antimicrobial agent containing the compound represented by the above Formula (1), Formula (2), or Formula (3), or a salt thereof, or a solvate thereof is also one of the present invention. The antimicrobial agent of the present invention usually contains the compound (I) as an active ingredient. The antimicrobial agent of the present invention can be used in various fields requiring antimicrobial properties, for example, the fields of medicines, foods and beverages, washing, and daily necessities. In one embodiment, the antimicrobial agent of the present invention can be provided in the form of a medicine.

A medicine containing the compound represented by the above Formula (1), Formula (2), or Formula (3), or a salt thereof, or a solvate thereof is also one of the present invention. The medicine of the present invention usually contains the compound (I) as an active ingredient.

The antimicrobial agent or the medicine of the present invention can be used for decolonization, disinfection, sterilization, or growth inhibition of microorganisms such as bacteria and fungi. Examples of microorganisms to be a target of the antimicrobial agent or the medicine include bacteria such as gram-positive bacteria and fungi such as yeast. The microorganism to be a target may be one type or two or more types.

Examples of the gram-positive bacteria include bacteria such as Mycobacterium bacteria (for example, Mycobacterium smegmatis), Staphylococcus aureus (for example, methicillin-resistant Staphylococcus aureus (MRSA)), Bacillus bacteria (for example, Bacillus subtilis), Micrococcus bacteria (for example, Micrococcus luteus), Kocuria bacteria (for example, Kocuria rhizophila), and vancomycin-resistant Enterococcus (VRE). Examples of the yeast include Candida yeasts (for example, Candida albicans).

In one embodiment, the antimicrobial agent or the medicine of the present invention can be used for preventing or treating infections. The medicine of the present invention may be, for example, a prophylactic or therapeutic agent for infections. Examples of the infection include bacterial infections and fungal infections. Examples of the bacterial infection include bacterial infections caused by the above gram-positive bacteria. Specific examples thereof include sepsis, meningitis, endocarditis, osteomyelitis, and pneumonia. Examples of the fungal infection include infections caused by yeasts (for example, Candida yeasts).

In the present specification, prevention means delaying or preventing the onset of the symptom or disease, or reducing the risk of a subject developing the symptom or disease. Treatment means alleviating the symptom or disease, preventing or delaying the worsening of the symptom or disease, or curing the symptom or disease.

The form of the antimicrobial agent or the medicine of the present invention is not limited, and may take a form that is usually used depending on the application. The compound or a salt thereof, or a solvate thereof can be used as it is or in various preparation forms.

The antimicrobial agent or the medicine may contain optional additives in addition to the compound or a salt thereof, or a solvate thereof. The additive is not limited as long as it is a pharmaceutically acceptable component, and examples thereof include carriers, excipients, binders, disintegrants, lubricants, antioxidants, and colorants. In one embodiment, the antimicrobial agent or the medicine of the present invention is provided as an antimicrobial composition or a pharmaceutical composition containing the compound or a salt thereof, or a solvate thereof, and an additive such as a pharmaceutically acceptable carrier.

The administration route when the antimicrobial agent or the medicine is administered to a subject is not limited, and may be either oral administration or parenteral administration (for example, transdermal, transmucosal, enteral, or injection administration). Examples of the dosage form for oral administration include liquid preparations, tablets, powders, fine granules, granules, dragees, capsules, suspensions, emulsions, and chewables. Examples of the dosage form for parenteral administration include injections, drops, and external preparations for skin (for example, patches, creams, and ointments).

The application subject of the antimicrobial agent or the medicine of the present invention (also referred to as an administration subject) is not limited. Examples thereof include mammals such as human, monkey, mouse, rat, rabbit, dog, cat, pig, cow, horse, sheep, and goat, and the subject is preferably human. In addition, examples of the application subject of the antimicrobial agent or the medicine include patients with infections, and subjects who desire or need prevention or treatment of infections.

The content of the compound (I) in the antimicrobial agent or the medicine of the present invention can be appropriately adjusted according to the use mode and the like. The content may be, for example, 0.0001 wt% or more and 100 wt% or less, preferably 0.001 wt% or more and 50 wt% or less in the antimicrobial agent or the medicine. The antimicrobial agent or the medicine may contain only one type of the compound (I) or two or more types thereof. When two or more types of compounds are used for the compound (I), the amount of the compound (I) is the total thereof.

When the compound (I) is administered to a subject, the dose thereof can be appropriately set according to, for example, the administration route, the application subject, the body weight of the subject, and the disease, but is not limited thereto. For example, when the compound (I) is administered to an adult, the compound (I) can be administered in a range of 0.001 mg/kg or more and 100 mg/kg or less per day. The number of administrations is, for example, once, twice, or three times per day.

### [Methods of prevention or treatment]

The present invention also encompasses a method for preventing or treating infections by administering the compound (I) described above. The present invention also encompasses use of the compound (I) for the prevention or treatment of infections. The infection is preferably a bacterial infection or a fungal infection.

The present invention also encompasses use of the compound (I) for the production of antimicrobial agents or medicines. In one embodiment, the antimicrobial agent or the medicine may be a prophylactic or therapeutic agent for infections.

### EXAMPLES

Hereinafter, examples more specifically disclosing the present invention will be described. Note that the present invention is not limited only to these examples.

### [Example 1]

### (Culturing of HV058 strain)

A Kitasatospora purpeofusca HV058 strain was seeded on the following medium and cultured. The Kitasatospora HV058 strain (Kitasatospora purpeofusca HV058 strain) has been deposited at the National Institute of Technology and Evaluation, Patent Microorganisms Depositary (292-0818, #122, 2-5-8, Kazusakamatari, Kisarazu-shi, Chiba, Japan) (accession date: 23, April, 2021, accession number: NITE BP-03475).

Medium specification: 1 L of ISP medium 4 to which 4% DIAION (registered trademark) HP20 (manufactured by Mitsubishi Chemical Corporation) was added
Culture conditions: 30°C, 7 days, 140 rpm
ISP medium 4 was prepared and used in the following composition.
Components (per 1 L): Soluble starch (10.000 g), K₂HPO₄ (1.000 g), MgSO₄·7H₂O (1.000 g), NaCl (1.000 g), (NH₄)₂SO₄ (2.000 g), CaCO₃ (2.000 g), FeSO₄·7H₂O (0.001 g), MnCl₂·7H₂O (0.001 g), ZnSO₄·7H₂O (0.001 g)
Final pH (25°C): 7.2 ± 0.2

After the above culturing, the culture solution containing bacterial cells was filtered to separate DIAION HP20. DIAION HP20 was washed with water in an amount of 5 times the volume thereof. Subsequently, to DIAION HP20, methanol in an amount of 5 times the volume thereof was added, and the substance adsorbed to DIAION HP20 was extracted for 1 hour. The methanol extract containing the substance adsorbed to DIAION HP20 was vacuum-dried to obtain a brown powder. The resulting brown powder was fractionated by ODS column chromatography (Cosmosil 140C₁₈-PREP (manufactured by Nacalai Tesque, Inc.)). A solution of the above powder in 10% methanol was passed through an ODS column. Then, elution was performed while the methanol content was increased stepwise by 10% from 10% methanol to finally 100% methanol. As a result, the target compound was detected in the 40% methanol fraction, and this fraction (referred to as fraction (A)) was analyzed by LC-MS.

The fraction (A) obtained above was vacuum-dried, and then the obtained powder was dissolved in methanol and purified under the following conditions by high-performance liquid chromatography (HPLC) to fractionate the watersoluble fraction.

Apparatus: High-performance liquid chromatograph system Prominence (manufactured by Shimadzu Corporation)
System controller: CBM-20 Alite
Pump: LC-20AD
Detector: SPD-M20A
Column: Cosmosil 5C₁₈MS-II 2.0 mm I.D. × 250 mm (manufactured by Nacalai Tesque, Inc.)

### Elution conditions:

Liquid A: H₂O 0.1% formic acid
Liquid B: Acetonitrile 0.1% formic acid
Concentration of liquid B: 10 vol% (0 to 5 min), 10 to 100 vol% (5 to 30 min), 100 vol% (30 to 40 min)
Flow rate: 0.2 mL/min
Temperature: 30°C
Detection: 210 nm

A target peak was detected at an elution time of 10 minutes under the above conditions.

### (Structural analysis of compound)

The molecular weight of the compound included in the target peak was larger by 5 Da than that of Hazimycin A (Non-Patent Literature 1). As a result of analyzing fragment ions by LC-MS/MS, the manner of dissociation from one side chain was the same as that of Hazimycin A, but m/z = 352.27 unique to the present compound was detected as a fragment ion dissociated from the other side chain. Thus, the presence of hydrazine serving as a side chain functional group in place of the isonitrile group was revealed.

It was found that the compound included in the target peak was a compound represented by the following Formula (1a-1) (compound (1a-1)).

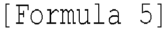

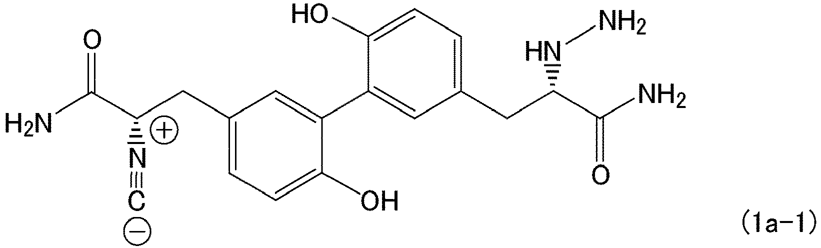

The antimicrobial activity of the compound (1a-1) was evaluated by the following method.

### (Antimicrobial test)

An antimicrobial test against the following bacteria and fungi was performed using a paper disc (6 mm, manufactured by ADVANTEC) containing the compound (1a-1).
(1) Gram-positive bacteria
   Mycobacterium smegmatis
   Bacillus subtilis
   Staphylococcus aureus
   Micrococcus luteus
(2) Gram-negative bacteria
   Escherichia coli
   Pseudomonas aeruginosa
(3) Yeast
   Candida albicans

An antimicrobial test was performed with a 10 µg/6 mm disc, using the culture solution of the above bacteria and fungi. The test was conducted in accordance with the method described in Koyama et al., Calpinactam, a new anti-mycobacterial agent, produced by Mortierella alpina FKI-4905, The Journal of Antibiotics (2010) 63, pages 183 to 186. The results (inhibitory zone (mm)) are shown in Table 1.

**[Table 1]**

| | | Inhibitory zone (mm) |
|---|---|---|
| Gram-positive bacteria | Mycobacterium smegmatis | 19 |
| | Bacillus subtilis | 14 |
| | Staphylococcus aureus | 23 |
| | Micrococcus luteus | 26 |
| Gram-negative bacteria | Escherichia coli | - |
| | Pseudomonas aeruginosa | - |
| Yeast | Candida albicans | 20 |

The compound (1a-1) showed an antimicrobial action against gram-positive bacteria and yeasts. The compound (1a-1) is useful as an active ingredient of an antimicrobial agent or a medicine.

## Claims

1. A compound represented by the following Formula (1), Formula (2), or Formula (3), or a salt thereof, or a solvate thereof: 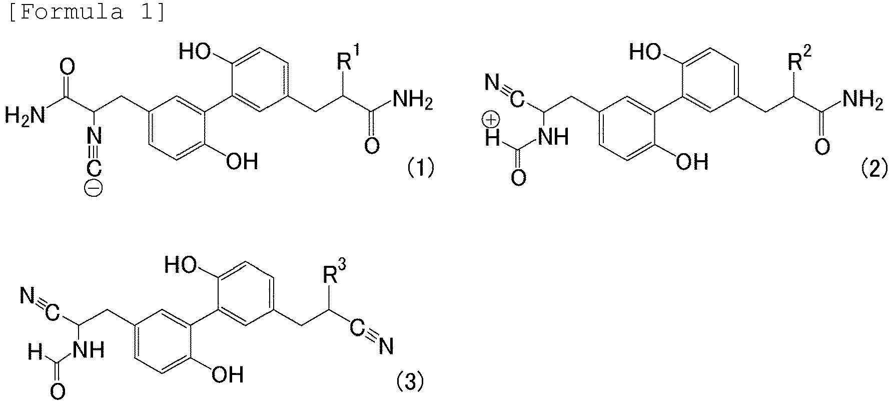 wherein R¹, R², and R³ are the same or different and represent a hydrazine group, an N-hydroxy-acetylamino group, or an N-hydroxy-formylamino group.

2. The compound or a salt thereof, or a solvate thereof according to claim 1, wherein R¹, R², and R³ are a hydrazine group.

3. A method for producing the compound or a salt thereof, or a solvate thereof according to claim 1 or 2, comprising:
a culture step of culturing a Kitasatospora purpeofusca HV058 strain (accession number NITE BP-03475); and
a collection step of collecting the compound or a salt thereof, or a solvate thereof according to claim 1 or 2 from a culture obtained in the culture step.

4. An antimicrobial agent comprising the compound or a salt thereof, or a solvate thereof according to claim 1 or 2.

5. A medicine comprising the compound or a salt thereof, or a solvate thereof according to claim 1 or 2.
